(19) Europäisches Patentamt
European Patent Office
Office européen des brevets

(11) Veröffentlichungsnummer: **0 005 474**
**B1**

(12) # EUROPÄISCHE PATENTSCHRIFT

(21) Anmeldenummer: **79101292.5**

(22) Anmeldetag: **30.04.79**

(51) Int. Cl.³: **C 07 C 143/665,**
C 07 C 97/24, C 09 B 1/22,
D 06 P 1/20

(54) **Anthrachinonverbindungen, Verfahren zu deren Herstellung sowie deren Verwendung zum Färben und Bedrucken von synthetischen Materialien**

(30) Priorität: **13.05.78 DE 2821148**

(43) Veröffentlichungstag der Anmeldung:
**28.11.79 Patentblatt 79/24**

(45) Bekanntmachung des Hinweises auf die Patenterteilung:
**12.11.80 Patentblatt 80/23**

(84) Benannte Vertragsstaaten:
**CH DE FR GB IT**

(56) Entgegenhaltungen:
**DE - A - 1 644 587**
**US - A - 2 174 245**

(73) Patentinhaber: **BAYER AG**
**ZENTRALBEREICH PATENTE, MARKEN UND LIZENZEN**
**D - 5090 LEVERKUSEN 1, BAYERWERK (DE)**

(72) Erfinder: **Kröck, Friedrich Wilhelm, Dr.**
**Gerstenkamp 12**
**D - 5000 Köln 80 (DE)**
**Neeff, Rütger, Dr.**
**Berta-von-Suttner-Strasse 22**
**D - 5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Anthrachinonverbindungen, Verfahren zu deren Herstellung sowie deren Verwendung zum Färben und Bedrucken von synthetischen Materialien

Gegenstand der vorliegenden Erfindung sind neue Anthrachinonfarbstoffe der Formel

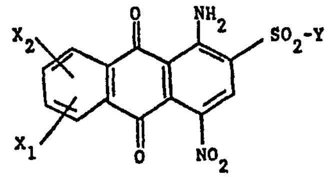

I

in welcher

Y eine Gruppe der Formeln —O—R oder

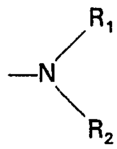

,

R gegebenenfalls substituiertes Aryl,

$R_1$ und $R_2$ Wasserstoff, gegebenenfalls substituiertes Alkyl, Cycloalkyl, Aralkyl oder Aryl, oder — gemeinsam — zusammen mit dem bindenden Stickstoffatom einen Ring und

$X_1$ und $X_2$ Wasserstoff, Halogen oder Nitro bedeuten, das Verfahren zu ihrer Herstellung, ihre Verwendung zum Färben von synthetischen Materialien sowie Zwischenprodukte — zur Herstellung dieser Farbstoffe — der Formel

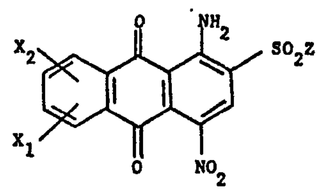

II

in welcher

$X_1$ und $X_2$ die oben genannte Bedeutung haben und

Z für Chlor oder Brom

steht und deren Herstellung.

Geeignete Arylreste R, $R_1$ und $R_2$ sind Naphthyl- und insbesondere Phenylreste, die in der Farbstoffchemie übliche Substituenten — beispielsweise solche der nachstehend angegebenen Art — enthalten können.

Geeignete Alkylreste $R_1$ und $R_2$ sind geradkettig oder verzweigt, weisen 1 bis 10 Kohlenstoffatome auf und können durch Halogen, Hydroxy, $C_1$- bis $C_4$-Alkoxy, Phenoxy, Amino, $C_1$- bis $C_4$-Alkylamino, $C_1$- bis $C_4$-Dialkylamino, Cyan, $C_1$- bis $C_4$-Alkoxy-carbonyl oder $C_5$- bis $C_6$-Cycloalkyl substituiert sein, sowie der Trifluoromethylrest. Geeignete Cycloalkylreste $R_1$ und $R_2$ sind solche mit 5 bis 6 Kohlenstoffatomen und können 1 bis 3 Substituenten, wie Halogen, Hydroxy, Cyan oder $C_1$- bis $C_4$-Alkyl, tragen.

Geeignete Aralkylreste $R_1$ und $R_2$ sind solche, die im Alkylenrest 1 bis 4 Kohlenstoffatome enthalten und deren Arylrest ein gegebenenfalls 1- bis 3-fach durch Halogen, $C_1$- bis $C_4$-Alkyl oder $C_1$- bis $C_4$-Alkoxy substituierter Naphthyl- und insbesondere Phenylrest ist.

Unter Halogen wird Fluor, Brom und vor allem Chlor verstanden. Bevorzugte Farbstoffe sind solche der Formel I, worin Y für OR steht, wobei R einen gegebenenfalls durch 1 bis 3 Reste aus der Reihe $C_1$- bis $C_4$-Alkyl, $C_1$- bis $C_4$-Alkoxy, Halogen wie insbesondere Fluor, Chlor, Brom, Methylmercapto, Nitro, Trifluormethyl, $C_5$- bis $C_6$-Cycloalkyl, Phenyl, Acetyl, Benzoyl, Carbomethoxy oder Carboxyl substituierten Phenylrest oder einen gegebenenfalls durch Methyl, Chlor oder Brom substituierten Naphthyl- oder 5,6,7,8-Tetrahydro-naphthylrest bedeutet.

Weiterhin sind solche Farbstoffe der Formel I bevorzugt, worin Y für $—NR_1R_2$ steht, wobei $R_1$ und $R_2$ Wasserstoff, gegebenenfalls durch Hydroxy, $C_1$- bis $C_4$-Alkoxy, Cyan oder Cyclohexyl substituiertes $C_1$- bis $C_4$-Alkyl, gegebenenfalls durch 1 bis 3 Methylreste oder durch Cyan substituiertes Cyclohexyl, gegebenenfalls 1- bis 3-fach durch Halogen, Methyl oder Methoxy substituiertes Benzyl, Phenethyl oder Naphthylmethyl oder durch 1 bis 3 Reste aus der Reihe $C_1$- bis $C_4$-Alkyl, $C_1$- bis $C_4$-Alkoxy, Chlor, Fluor, Brom, Nitro, Trifluormethyl, $C_5$- bis $C_6$-Cycloalkyl, Phenyl oder Acetyl substituiertes Phenyl oder gegebenenfalls durch Methyl, Chlor oder Brom substituiertes Naphthyl oder 5,6,7,8-Tetrahydronaphthyl bedeuten und solche in denen $R_1$ und $R_2$ zusammen mit dem bindenden Stickstoffatom für

$-(CH_2)_4-$, $-(CH_2)_5-$ oder $-(CH_2)_2-O-(CH_2)_2-$ stehen.

Aus der letzten Gruppe sind ganz besonders diejenigen Farbstoffe bevorzugt, in denen einer der Reste $R_1$ und $R_6$ für Wasserstoff steht oder in denen $R_1$ und $R_2$ beide für gegebenenfalls wie zuvor substituiertes $C_1$- bis $C_6$-Alkyl stehen.

Vorzugsweise stehen $X_1$ und $X_2$ für Wasserstoff.

"Sperrige" Reste befinden sich vorzugsweise in solchen Positionen, wo sie keine sterische Hinderung bewirken.

Die neuen Farbstoffe werden beispielsweise erhalten, indem man Anthrachinonsulfonsäurehalogenide der Formel II mit Phenolen der Formel ROH, in welcher R die obengenannte Bedeutung hat, oder mit Aminen der Formel

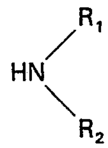 ,

in welcher $R_1$ und $R_2$ die obengenannte Bedeutung haben, in an sich bekannter Weise umsetzt.

Beispiele der als Ausgangsmaterial verwendeten Anthrachinonsulfonsäurehalogenide II sind insbesondere die entsprechenden Anthrachinonsulfonsäurechloride, wie die folgenden:

1-Amino-4-nitro-anthrachinon-2-sulfonsäurechlorid,
1-Amino-5-chlor-anthrachinon-2-sulfonsäurechlorid,
1-Amino-4-nitro-6-chlor-anthrachinon-2-sulfonsäurechlorid,
1-Amino-4-nitro-7-chloranthrachinon-2-sulfonsäurechlorid,
1-Amino-4-nitro-6,7-dichlor-anthrachinon-2-sulfonsäurechlorid,
1-Amino-4-nitro-6-fluor-anthrachinon-2-sulfonsäurechlorid,
1-Amino-4-nitro-7-fluor-anthrachinon-2-sulfonsäurechlorid,
1-Amino-4-nitro-6,7-difluor-anthrachinon-2-sulfonsäurechlorid,
1-Amino-4,5-dinitro-anthrachinon-2-sulfonsäurechlorid,
1-Amino-4,6-dinitro-anthrachinon-2-sulfonsäurechlorid,
1-Amino-4,7-dinitro-anthrachinon-2-sulfonsäurechlorid,
1-Amino-4,8-dinitro-anthrachinon-2-sulfonsäurechlorid,

sowie die entsprechenden Sulfonsäurebromide, insbensondere

1-Amino-4-nitro-anthrachinon-2-sulfonsäurebromid.

Beispiele der als Ausgangsmaterialien verwendeten Phenole ROH sind die folgenden:

Phenol, p-Chlorphenol, m-Chlorphenol, o-Chlorphenol, p-Bromphenol, o-, m-, p-Kresol, 2,4-Dichlorphenol, 3,4-dimethylphenol, 3-Chlor-6-methylphenol, p-Methylmercaptophenol, m-Methoxyphenyl, p-Methoxyphenol, p-Acetylphenol, 3-Hydroxybenzoesäure, 4-Hydroxybenzoesäuremethylester, p-Trifluormethylphenol, 3-Nitrophenol, 4-Cyclohexyl-phenol, 4-Phenylphenol, 4-Hydroxybenzophenon, $\alpha$-Naphthol, $\beta$-Naphthol und 5,6,7,8-Tetrahydro-1-naphthol.

Beispiele der als Ausgangsmaterialien verwendeten Amine

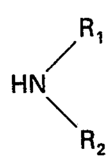

Ammoniak, Methylamin, Ethylamin, Isopropylamin, n-Propylamin, n-Butylamin, Isobutylamin, tert.-Butylamin, n-Pentylamin, n-Hexylamin, Decylamin, Dimethylamin, Diethylamin, Dipropylamin, Dibutylamin, 2-Hydroxy-ethylamin, 2-Methoxy-ethylamin, 2-Ethoxy-ethylamin, 2-Butoxy-ethylamin, 2-Cyan-ethylamin, 2-Chlor-ethylamin, 2-Methylamino-ethanol, Bis-(2-hydroxyethyl)-amin, 1-Amino-2-propanol, 2,3-Dihydroxy-propylamin, 2-Phenoxy-ethylamin, 1,2-Diamino-ethan, N,N-Dimethyl-1,2-diaminoethan, 3-Hydroxy-propylamin, 3-Methoxy-propylamin, N,N-Dimethyl-1,3-diamino-propan, Aminoessigsäure-methylester, Aminoessigsäureethylester, Cyclopentylamin, Cyclohexylamin, 4-Cyancyclohexylamin, 4-Methyl-cyclohexylamin, 3-Methyl-cyclohexylamin, 3,3,5-Trimethyl-cyclohexylamin, N-Methyl-cyclohexylamin, Amino-methyl-cyclohexan, Benzylamin, 4-Chlor-benzylamin, 3- und 4-Methyl-benzylamin, 3- und 4-Methoxy-benzylamin, $\beta$-Phenethylamin, Anilin, 4-Chlor-anilin, 3-Chlor-anilin, 2-Chlor-anilin, 4-Brom-anilin, 2-Methyl-anilin, 3-Methyl-anilin, 4-Methyl-anilin, 2,4-Dimethylanilin, 2,4-Dichlor-anilin, 2,5-Dichloranilin, 4-Hydroxy-anilin, 4-Methoxy-anilin, 3-Methoxy-anilin, Anthranilsäure, Anthranilsäure-methyl- und -ethylester, 4-Trifluormethyl-anilin, 3-Trifluormethyl-anilin,

3-Nitranilin, 4-Cyclohexyl-anilin, 4-Amino-diphenyl, $\alpha$-Naphthylamin, 1-Amino-5,6,7,8-tetrahydro-naphthalin, 2-Amino-5,6,7,8-tetrahydro-naphthalin, 1-Amino-1,2,3,4-tetrahydro-naphthalin, 2-Amino-1,2,3,4-tetrahydro-naphthalin.

Die Umsetzung der Anthrachinonsulfonsäurehalogenide II mit Phenolen der Formel ROH oder Aminen der Formel

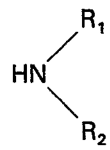

kann in wäßrigem Medium oder in einem organischen Lösungsmittel durchgeführt werden, wobei man im allgemeinen unter Zusatz eines säurebindenden Mittels arbeitet. Geeignete säurebindende Mittel sind anorganische Basen wie beispielsweise Oxide oder Hydroxide von Alkali- oder Erdalkalimetallen, wie Natriumhydroxid, Kaliumhydroxid oder Calciumoxid, Alkalimetallsalze schwacher Säuren, wie Natriumcarbonat, Kaliumcarbonat, Natriumacetat oder Kaliumacetat, oder organische Basen, wie z.B. Trimethylamin, Triethylamin oder Benzyltrimethyl-ammoniumhydroxid. Erfolgt die Umsetzung mit Aminen der Formel

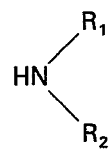

so kann gegebenenfalls auf ein zusätzliches säurebindendes Mittel verzichtet und as Amin im Ueberschuß eingesetzt werden.

Als organische Lösungsmittel seien zum Beispiel genannt: Toluol, Chlorbenzol, 1,2-Dichlorbenzol, Nitrobenzol, Dioxan, Tetrahydrofuran, Dimethylformamid, Dimethylsulfoxid, Tetramethylensulfon (Sulfolan), Pyridin, Diglykolmonomethylether, Glykoldimethylether, Aceton und Methylethylketon.

Die Umsetzung kann bei Raumtemperatur oder bei höheren bzw. niedrigeren Temperaturen erfolgen, z.B. zwischen −20 und +100°C, vorzugsweise bei 0 bis 60°C.

Die neuen Farbstoffe der Formel I, sowie Gemische derselben untereinander und Gemische mit geeigneten bekannten Farbstoffen eignen sich ausgezeichnet zum Färben und Bedrucken von synthetischen Fasermaterialien aus Celluloseester, Polyamiden, Polyurethanen, Polyacrylnitrilen und aromatischen Polyestern, insbesondere Polyethylenglykolterephthalaten nach herkömmlichen Färbeverfahren.

Das Ausfärben bzw. Bedrucken kann nach an sich für das Färben aus wäßriger Flotte bekannten Verfahren sowohl mit den reinen Farbstoffen, als auch mit Mischungen aus zwei oder mehreren Farbstoffen erfolgen. Es ist dabei verteilhaft, die Farbstoffe bzw. die Farbstoffmischungen vor der Verwendung nach den gebräuchlichen Methoden in einen Zustand feiner Verteilung zu bringen.

Darüber hinaus eignen sich die neuen Farbstoffe zum Färben der genannten Faserarten aus mit Wasser nicht mischbaren organischen Lösungsmitteln nach dem Ausziehverfahren, wie es beispielsweise in den Britischen Patentschriften 1 314 022 und 1 284 670 (=US 3 792 971) beschrieben ist. Bevorzugtes Lösungsmittel ist dabei Tetrachlorethylen.

Weiterhin eignen sich die neuen Farbstoffe vorzüglich zum Färben von Mischgeweben aus synthetischen und natürlichen Fasermaterialien, vorzugsweise solchen aus Polyester und Cellulose (besonders Baumwolle) und Polyester und Wolle.

Man kann die neuen wasserunlöslichen Farbstoffe auch zur Spinnfärbung von Polyamiden, Polyestern und Polyolefinen verwenden.

Die auf den genannten Faserarten erzeugten Färbungen zeichnen sich durch gute Allgemeinechtheiten aus.

Im Vergleich zu den konstitutionell nächstliegenden bekannten Farbstoffen gemäß DOS 16 44 578 und DAS 1 176 777 zeigen die erfindungsgemäßen Farbstoffe der Formel I den überraschenden Vorteil des besseren Sublimierechtheit der Polyesterfärbungen bzw. des besseren Aufbauvermögens beim Färben von Cellulosetriacetatfasern. Dieser Effekt war in keiner Weise vorhersehbar — auch nicht im Hinblick auf die sehr allgemein gehaltene Lehre gemäß US—PS 2174245.

Die Anthrachinonsulfonsäurehalogenide der Formel II, die ebenfalls Gegenstand der vorliegenden Erfindung sind, werden beispielsweise erhalten, indem man Anthrachinonsulfonsäuren der Formel

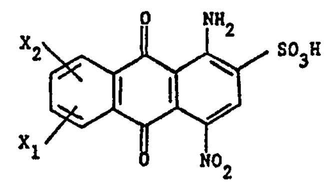

III

in welcher

$X_1$ und $X_2$ die oben genannte Bedeutung haben,

oder deren Salze mit entsprechenden anorganischen Säurehalogeniden behandelt, beziehungsweise indem man die so erhaltenen Sulfonsäurechloride in an sich bekannter Weise in die Sulfonsäurebromide überführt.

Die als Ausgangsmaterial verwendeten Anthrachinonsulfonsäuren III sind zum Teil bekannt (vgl. DOS 1 906 834 bzw. CH-Anmeldung 2468—68 und DAS 1 176 777, Beispiel 7) beziehungsweise können nach bekannten Verfahren hergestellt werden.

Geeignete anorganische Säurehalogenide sind zum Beispiel Chlorsulfonsäure, Thionylchlorid, Phosphortrichlorid, Phosphoroxychlorid, Phosphorpentachlorid, Thionylbromid, Phosphortribromid, Phosphorpentabromid und Phosphoroxybromid.

Zur Herstellung der Sulfonsäurechloride gemäß Formel II wird Phosphoroxychlorid bevorzugt.

Die Umsetzung der Anthrachinonsulfonsäuren III mit anorganischen Säurehalogeniden kann nach prinzipiell bekannten Verfahren in einem geeigneten Lösungsmittel oder im überschüssigen anorganischen Säurehalogenid selbst erfolgen.

Als Beispiele für geeignete Lösungsmittel seien insbesondere genannt:

Toluol, Chlorbenzol, 1,2-Dichlorbenzol, 1,2,4-Trichlorbenzol, Nitrobenzol, Dioxan, Tetrahydrofuran, Dimethylformamid, Dimethylsulfoxid, Tetramethylensulfon (Sulfolan), Glykoldimethylether, Chloroform und 1,2-Dichlorethan.

Die Umsetzung kann je nach Reaktivität des eingesetzten anorganischen Säurehalogenids bei Raumtemperatur oder bei höheren beziehungsweise niedrigeren Temperaturen erfolgen, z.B. zwischen 0 und 150°C, vorzugsweise bei 40 bis 120°C. Dabei geht man beispielsweise so vor, daß man das überschüssige anorganische Säurehalogenid vorlegt, das gut getrocknete Alkalisalz der umzusetzenden Sulfonsäure III bei Raumtemperatur einträgt und die Mischung auf die erforderliche Temperatur bringt oder man trägt das Alkalisalz der Sulfonsäure III bei Reaktionstemperatur in das vorgelegte anorganische Säurechlorid ein. Nach Beendigung der Reaktion kann das gebildete Sulfonsäurehalogenid in verschiedener Weise isoliert werden. Man kann zum Beispiel das Reaktionsgemisch, gegebenenfalls nachdem man das anorganische Säurehalogenid ganz oder zum Teil abdestilliert hat, abkühlen und das direkt oder nach Verdünnen mit einem geeigneten inerten Lösungsmittel auskristallisierte Sulfonsäurehalogenid abtrennen. Eine andere Möglichkeit besteht darin, daß man das Reaktionsgemisch, gegebenenfalls nachdem man das anorganische Säurehalogenid ganz oder zum Teil abdestilliert hat, abkühlt und auf Eiswasser austrägt, bis zur völligen Zersetzung des überschüssigen anorganischen Säurehalogenids bei maximal 25°C verrührt und dann das ausgefallene Sulfonsäurehalogenid der Formel II abtrennt. Wird die Umsetzung in einem inerten Lösungsmittel durchgeführt, so kann es im Falle eines mit Wasser nicht mischbaren Lösungsmittels zweckmäßiger sein, das Lösungsmittel zum Teil oder ganz durch Destillation gegebenenfalls im Vakuum zu entfernen und dann das Sulfonsäurehalogenid durch Einrühren in Wasser oder durch Zusatz eines geeigneten anderen Lösungsmittels zur Kristallisation zu bringen.

Wird die Umsetzung in Chlorsulfonsäure durchgeführt, so kann man dabei vorgehen wie in der DAS 1 271 284 beschrieben und die Sulfonsäuren oder deren Salze in überschüssiger Chlorsulfonsäure bei Raumtemperatur solange verrühren, bis das Ausgangsmaterial vollständig umgesetzt ist. Günstiger ist es jedoch meistens, wenn man die Sulfonsäurechlorid-Herstellung in einem Gemisch aus Chlorsulfonsäure und Thionylchlorid entsprechend den Angaben der DOS 2 356 776 durchführt, oder in einem Gemisch aus Thionylchlorid und Schwefeltrioxid entsprechend den Angaben der DOS 26 35 281 (= BE 8 57 498) arbeitet.

Zur Herstellung des Sulfonsäurebromids kann man auch so vorgehen, daß man das Sulfonsäurechlorid in an sich bekannter Weise in das Sulfonsäurebromid überführt, beispielsweise durch Behandlung mit einem Alkali- oder Erdalkalibromid, insbesondere mit Lithium- oder Calciumbromid, in einem geeigneten wasserfreien organischen Lösungsmittel, wie insbesondere Aceton oder einem Alkohol, wie z.B. Ethanol, bei Raumtemperatur oder gegebenenfalls bis zum Siedepunkt der Reaktionsmischung erhöhter Temperatur (vgl. z.B. Organikum. S. 197—198; 5. Aufl.).

Beispiel 1

185 g gut getrocknetes 1-amino-4-nitro-anthrachinon-2-sulfonsaures Natrium, hergestellt nach der Vorschrift in DOS 1 906 834, Bsp. 1 oder Bsp. 2, trägt man portionsweise in 1 240 g (= 740 ccm) Phosphoroxychlorid bei 100°C ein. Man hält das Gemisch unter Rühren bei 100°C, bis das Ausgangsmaterial lt. Dünnschichtchromatogramm nach ca. 2 Stunden restlos verschwunden und eine Lösung entstanden ist. Nach dem Abkühlen gießt man das Reaktionsgemisch in 7 l Eiswasser ein, so daß die

Temperatur 20°C nicht übersteigt, ruhrt das Gemisch, bis das Phosphoroxychlorid nach etwa 1 Stunde völlig zersetzt und das Sulfonsäurechlorid der Formel

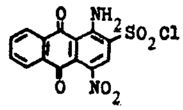

ausgefallen ist, saugt ab und wäscht mit kaltem Wasser neutral. Das Produkt wird im Vakuum über Natriumhyroxid getrocknet. Ausbeute: 174,5 g, entsprechend 95% d. Th. Schmp. ca. 250°C (Zers.).

Analyse: $C_{14}H_7ClN_2O_6S$ (366,7)

| | | | | | |
|---|---|---|---|---|---|
| Berechnet: | C 45,85 | H 1,92 | N 7,64 | Cl 9,68 | S 8,74 |
| Gefunden: | 46,1 | 1,8 | 7,6 | 9,9 | 8,8 |

Statt das Reaktionsgemisch in Wasser einzugießen, kann man auch wie folgt aufarbeiten:

a) Man kühlt die Lösung im Eisbad für 2 Stunden auf 0 bis 5°C und saugt dann die ausgefallenen Kristalle auf einer Glasfritte ab, trägt sie in Eiswasser ein, rührt 1Stunde, saugt erneut ab, wäscht neutral und trocknet im Vakuum über Natriumhydroxid. Ausbeute: 132 g, entsprechend 72% d. Th. Schmp. ca. 250°C (Zers.).

b) Aus dem erhaltenen Reaktionsgemisch werden ca 400 ccm Phosphoroxychlorid im Vakuum abdestilliert. Der Rückstand wird im Eisbad für 2 Stunden auf 0 bis 5°C gekühlt. Dann werden die ausgefallenen Kristalle scharf abgesaugt, 1 Stunde bei maximal 20°C mit 1,5 l Wasser verrührt, erneut abgesaugt, mit kaltem Wasser neutral gewaschen und wie oben getrocknet. Ausbeute: 173 g, entsprechend 94% d. Th.

Das zurückgewonnene Phosphoroxychlorid kann erneut zur Herstellung des Sulfonsäurechlorids verwendet werden.

c) Aus dem erhaltenen Reaktionsgemisch werden wie zuvor ca. 400 ccm Phosphoroxychlorid abdestilliert. Der Rückstand wird in 2,5 l Eiswasser eingegossen und 1 Stunde bei max. 20°C gerührt, bis das Phosphoroxychlorid zersetzt ist. Das ausgefallene Sulfonsäurechlorid wird wie zuvor abgesaugt, gewaschen und getrocknet. Ausbeute: 176 g, entsprechend 96% d. Th.

Das zurückgewonnene Phosphoroxychlorid kann erneut zur Herstellung des Sulfonsäurechlorids verwendet werden.

d) In ähnlicher Ausbeute und Reinheit wird das Sulfonsäurechlorid auch erhalten, wenn man statt Phosphoroxychlorid Phosphortrichlorid oder Phosphorpentachlorid verwendet.

Beispiel 2

37 g gut getrocknetes 1-amino-4-nitro-anthrachinon-2-sulfonsaures Natrium suspendiert man in 300 ccm Toluol, erhitzt auf 100°C und tropft bei dieser Temperatur 48 g Phosphoroxychlorid dazu. Man hält ca. 20 Stunden auf dieser Temperatur, filtriert das nicht umgesetzt, ungelöste Ausgangsmaterial ab, destilliert dann das Toluol im Vakuum ab und rührt den Rückstand in 500 ccm Wasser bei max. 20°C ca. 1 Stunde aus. Dann wird das Sulfonsäurechlorid abgesaugt, neutralgewaschen und wie in Beispiel 1 getrocknet. Ausbeute: 33 g, entsprechend 90% d. Th.

Wie Schmelz- und Mischschmelzpunkt zeigen, erhält man dasselbe Produkt wie in Beispiel 1 beschrieben.

In ähnlicher Ausbeute und Reinheit wird das Sulfonsäurechlorid erhalten, wenn man statt Toluol Chlorbenzol, 1,2-Dichlorbenzol, 1,2,4-Trichlorbenzol, Nitrobenzol, Dioxan, Tetrahydrofuran, Dimethylformamid, Dimethylsulfoxid, Tetramethylensulfon, Glykoldimethylether, Chloroform oder 1,2-Dichlorethan verwendet.

Beispiel 3

In 76 g (= 43 ccm) Chlorsulfonsäure trägt man 18,5 g gut getrocknetes 1-amino-4-nitro-anthrachinon-2-sulfonsaures Natrium ein und tropft bei 20 bis 25°C in 60 Minuten 22,5 g (= 13,5 ccm) Thionylchlorid dazu. Wenn nach etwa 3 Stunden das Ausgangsmaterial restlos umgesetzt ist, gießt man das Gemisch auf 750 ccm Eiswasser, saugt das ausgefallene Sulfonsäurechlorid ab, wäscht es neutral und trocknet wie in Beispiel 1 beschrieben. Ausbeute: 17 g, entsprechend 92% d. Th.

In ähnlich guter Ausbeute und Reinheit wird das Sulfonsäurechlorid geonnen, wenn man entsprechend der Vorschrift in DOS 2 635 281 (= BE 857 498) in überschüssigem Thionylchlorid in Gegenwart von katalytischen Mengen Schwefeltrioxid oder Chlorsulfonsäure arbeitet.

Beispiel 4

55,5 g Phosphorpentabromid und 18,5 g gut getrocknetes 1-amino-4-nitro-anthrachinon-2-sulfonsaures Natrium werden in 200 ccm Tetrahydrofuran eingetragen. Man hält des Gemisch unter

Rühren bei 100°C, bis das Ausgangsmaterial lt. Dünnschichtchromatogramm umgesetzt ist, kühlt ab und gießt die Reaktionsmischung in 1 l Eiswasser ein. Nach 1-stündigem Rühren bei 0—5°C saugt man ab, wäscht neutral und trocknet. Ausbeute: 17 g, entsprechend 82% d. Th. des Produktes der Formel

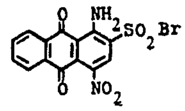

dessen Struktur durch Analysen und Massenspektrum bestätigt wird. Statt mit Phosphorpentabromid kann die Reaktion auch mit Phosphortribromid, Phosphoroxybromid oder Thionylbromid durchgeführt werden. Ebenso kann statt Tetrahydrofuran auch Dioxan, Toluol, Chlorbenzol oder Dimethylformamid verwendet werden. Bei Verwendung von Toluol oder Chlorbenzol muß das Lösungsmittel vor Eintragen in Wasser abdestilliert werden.

Statt aus der Sulfonsaure kann das Sulfonsäurebromid auch durch Chloraustauschreaktion aus dem Sulfonsäurechlorid, das nach einer der vorstehenden Beispiele erhalten wurde, erhalten werden. Die Reaktion kann z.B. mit Lithiumbromid oder Calciumbromid in Aceton oder Ethanol durchgeführt werden.

Beispiele 5—21

Entsprechend der Vorschriften der Beispiele 1 bis 4 können auch die in Tabelle 1 aufgeführten Sulfonsäurechloride und -bromide hergestellt werden, deren Struktur durch Analysen und Massenspektren einerseits, sowie durch ihre Reaktionsprodukte mit Phenolen und Aminen andererseits bestätigt wird.

TABELLE 1

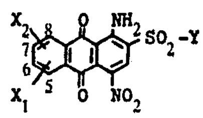

| Bsp.-Nr. | Y | $X_1$ | $X_2$ |
|---|---|---|---|
| 5 | Cl | 5-Cl | H |
| 6 | Cl | 6-Cl | H |
| 7 | Cl | 7-Cl | H |
| 8 | Cl | 8-Cl | H |
| 9 | Cl | 5-Cl | 8-Cl |
| 10 | Cl | 6-Cl | 7-Cl |
| 11 | Cl | 6-F | H |
| 12 | Cl | 7-F | H |
| 13 | Cl | 6-F | 7-F |
| 14 | Cl | 5-$NO_2$ | H |
| 15 | Cl | 6-$NO_2$ | H |
| 16 | Cl | 7-$NO_2$ | H |
| 17 | Cl | 8-$NO_2$ | H |
| 18 | Cl | 5-Br | H |
| 19 | Br | 6-Cl | H |
| 20 | Br | 5-Cl | 8-Cl |
| 21 | Br | 5-$NO_2$ | H |

Beispiel 22

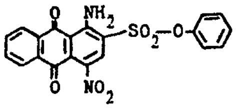

In 400 ccm Chlorbenzol trägt man 59 g Phenol und 47,5 g trockenes Kaliumcarbonat ein, erhitzt auf 100°F und destilliert durch Anlegen eines Vakuums ca. 40 bis 50 ccm Chlorbenzol ab. Nach Abkühlen des Gemisches auf 25°C gibt man 91,5 g 1-Amino-4-nitro-anthrachinon-2-sulfonsäurechlorid (Beispiel 1) hinzu. Nach Abklingen der exothermen Reaktion rührt man noch 30 Minuten, verdünnt dann mit 500 ccm Methanol und saugt nach weiteren 30 Minuten ab. Man wäscht mit Methanol und dann mit Wasser und trocknet bei 60°C. Ausbeute: 9,2 g, entsprechend 86% d. Th.

Analyse: $C_{20}H_{12}N_2O_7S$ (424.4)

| | | | | |
|---|---|---|---|---|
| Berechnet: | C 56,60 | H 2,85 | N 6,60 | S 7,56 |
| Gefunden: | 56,5 | 2,7 | 6,3 | 7,7 |

Im vorstehenden Beispiel kann das als säurebindendes Mittel verwendete kaliumcarbonat auch durch Natriumhydroxid, Kaliumhydroxid, Calciumoxid, Natriumcarbonat, Natriumhydrogencarbonat, Natriumacetat oder Kaliumacetat ersetzt werden. Dabei kann der Sulfonsäure-arylester in ähnlich guter Ausbeute und Reinheit erhalten werden. Aehnlich gute Ausbeute und Reinheit erzielt man auch mit organischen Basen, mit Trimethylamin, Triethylamin, Pyridin oder Benzyltrimethyl-ammoniumhydroxid.

Ersetzt man im vorstehenden Beispiel das Chlorbenzol durch die gleiche Menge Tetrahydrofuran, so kann dasselbe Reaktionsprodukt in ähnlich guter Ausbeute und Reinheit isoliert werden. Auch Toluol, 1,2-Dichlorbenzol, Nitrobenzol, Chloroform, 1,2-Dichlorethan, Dioxan, Dimethylformamid, Dimethylsulfoxid, Sulfolan, Pyridin, Diglykolmonomethylether, Glykoldimethylether, Aceton und Methylethylketon können mit demselben Erfolg als Lösungsmittel verwendet werden.

Mit dem erhaltenen Farbstoff erzielt man auf synthetischen Fasermaterialien aus Celluloseestern, Polyamiden, Polyurethanen, Polyacrylnitrilen und insbesondere Polyestern rotstichige Gelbtöne mit guten Echtheiten.

Beispiele 23—120

Analog wie in Beispiel 22 beschriben, werden die in Tabelle 2 aufgeführten Farbstoffe hergestellt, die auf Geweben oder Gewirken aus Polyester-, Triacetat-, Polyamid-, Polyurethan- oder Polyolefinfasern die angegebenen Nuancen ergeben.

TABELLE 2

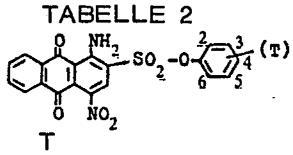

1—3

| Bsp.–Nr. | T | Farbton |
|---|---|---|
| 23 | 2-$CH_3$ | Gelb |
| 24 | 3-$CH_3$ | Gelb |
| 25 | 4-$CH_3$ | Gelb |
| 26 | 2,4-Di-$CH_3$ | etw. rotstichig Gelb |
| 27 | 2-Cl | rotst. Gelb |
| 28 | 3-Cl | Gelb |
| 29 | 4-Cl | Gelb |
| 30 | 2,4-Di-Cl | rotst. Gelb |
| 31 | 2,5-Di-Cl | rotst. Gelb |
| 32 | 2,6-Di-Cl | Gelb |
| 33 | 2,4,5-Tri-Cl | Gelb |
| 34 | 2,4,6-Tri-Cl | Gelb |
| 35 | 2-$NO_2$ | rotst. Gelb |
| 36 | 3-$NO_2$ | rotst. Gelb |
| 37 | 4-$NO_2$ | rotst. Gelb |
| 38 | 2-$NO_2$-4-Cl | Gelb |
| 39 | 3-$No_2$-4-Cl | Gelb |
| 40 | 2-Cl-5-$NO_2$ | Gelb |
| 41 | 3-Cl-4-$NO_2$ | Gelb |
| 42 | 2-$CH_3$-6-Cl | Gelb |
| 43 | 2-$CH_3$-5-Cl | Gelb |
| 44 | 2-$CH_3$-4-Cl | rotst. Gelb |
| 45 | 2-$CH_3$-5-$NO_2$ | Gelb |
| 46 | 2-Cl-5-$CH_3$ | Gelb |
| 47 | 3-$CH_3$-4-Cl | rotst. Gelb |
| 48 | 3-$CH_3$-4-$NO_2$ | Gelb. |
| 49 | 2-$C_2H_5$ | rotst. Gelb |
| 50 | 2,3-Di-$CH_3$ | Gelb |
| 51 | 3,4-Di-$CH_3$ | Gelb |

TABELLE 2 (Fortsetzung)

| Bsp.–Nr. | T | Farbton |
|---|---|---|
| 52 | 2,6-Di-$CH_3$ | Gelb |
| 53 | 3,5-Di-$CH_3$ | Gelb |
| 54 | 3,5-Di-$CH_3$-4-Cl | Gelb |
| 55 | 2,5-Di-$CH_3$ | Gelb |
| 56 | 2-Isopropyl | rotst. Gelb |
| 57 | 3-$CH_3$-5-$C_2H_5$ | rotst. Gelb |
| 58 | 2,3,5-Tri-$CH_3$ | Gelb |
| 59 | 2-Isobutyl | Gelb |
| 60 | 2-tert.-Butyl | Gelb |
| 61 | 4-tert.-Butyl | rotst. Gelb |
| 62 | 3-$CH_3$-5-isopropyl | rotst. Gelb |
| 63 | 4-Isobutyl | rotst. Gelb |
| 64 | 4-Isooctyl | Gelb |
| 65 | 2,4-Di-isobutyl | Gelb |
| 66 | 2-Cyclohexyl | Gelb |
| 67 | 4-Cyclohexyl | rotst. Gelb |
| 68 | 4-Cyclopentyl | rotst. Gelb |
| 69 | 2-Phenyl | Gelb |
| 70 | 3-Phenyl | Gelb |
| 71 | 4-Phenyl | Gelb |
| 72 | 2-Brom-4-phenyl | Gelb |
| 73 | 2-Benzyl | Gelb |
| 74 | 4-Benzyl | rotst. Gelb |
| 75 | 4-(2-Phenyl-propyl-2)- | Gelb |
| 76 | 2-$OCH_3$ | Gelb |
| 77 | 3-$OCH_3$ | Gelb |
| 78 | 4-$OCH_3$ | rotst. Gelb |
| 79 | 2-$OC_2H_5$ | Gelb |
| 80 | 4-$OC_2H_5$ | rotst. Gelb |
| 81 | 2-Isopropyloxy | Gelb |
| 82 | 4-Isopropyloxy | rotst. Gelb |
| 83 | 4-n-Butyloxy | rotst. Gelb |

TABELLE 2 (Fortsetzung)

| Bsp.-Nr. | T | Farbton |
|---|---|---|
| 84 | 3-F | Gelb |
| 85 | 4-F | Gelb |
| 86 | 3-$CF_3$ | Gelb |
| 87 | 4-$CF_3$ | rotst. Gelb |
| 88 | 3-Br | Gelb |
| 89 | 4-Br | rotst. Gelb |
| 90 | 4-OPh | rotst. Gelb |
| 91 | 4-O-Cyclohexyl | rotst. Gelb |
| 92 | 3-CO–$CH_3$ | Gelb |
| 93 | 4-CO–$CH_3$ | Gelb |
| 94 | 4-CO–Ph | Gelb |
| 95 | 3-$COOCH_3$ | Gelb |
| 96 | 4-$COOCH_3$ | Gelb |
| 97 | 3-COOH | Gelb |
| 98 | 3-CN | Gelb |
| 99 | 4-CN | rotst. Gelb |
| 100 | 4-$SO_2$–$CH_3$ | Gelb |
| 101 | 4-$SO_2$–Ph | Gelb |
| 102 | 4-$SCH_3$ | rotst. Gelb |
| 103 | 3-$CH_3$-4-$SCH_3$ | Gelb |
| 104 | 2,6-Di-$CH_3$-4-$SCH_3$ | Gelb |

TABELLE 2 (Fortsetzung)

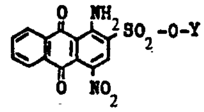

| Bsp.–Nr. | Y | Farbton |
|---|---|---|
| 105 | 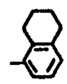 | Gelb |
| 106 | 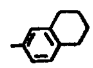 | Gelb |
| 107 | 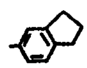 | Gelb |
| 108 | 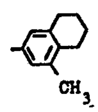 | Gelb |
| 109 | 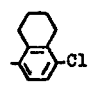 | Gelb |
| 110 | 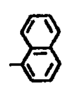 | rotst. Gelb |
| 111 | 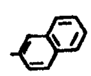 | rotst. Gelb |
| 112 | 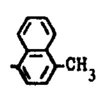 | Gelb |
| 113 | 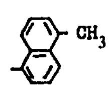 | Gelb |
| 114 | 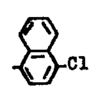 | Gelb |
| 115 | 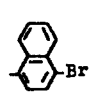 | Gelb |

TABELLE 2 (Fortsetzung)

| Bsp.-Nr. | Y | Farbton |
|---|---|---|
| 116 | 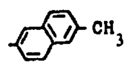 | rotst. Gelb |
| 117 | 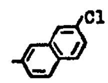 | Gelb |
| 118 | 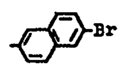 | Gelb |
| 119 | 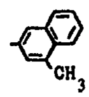 | Gelb |
| 120 | 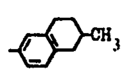 | Gelb |

Beispiel 121

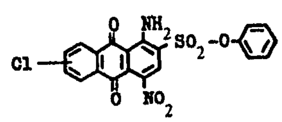

Ein Gemisch aus 40 ccm Chlorbenzol, 5,9 g Phenol und 4,75 g trockenem Kaliumcarbonat destilliert man bei 100°C im Vakuum an (einige ccm Chlorbenzol werden abdestilliert). Nach dem Abkühlen auf 25°C trägt man 10,3 g eines Gemisches aus 1-Amino-4-nitro-6-chlor- und -7-chlor-anthrachinon-2-sulfonsäurechlorid ein, wobei die Temperatur um etwa 10°C ansteigt. Man rührt noch 30 Min., verdünnt das Reaktionsgemisch dann mit 50 ccm Methanol, saugt nach 30 Min. ab, wäscht mit Methanol und dann mit Wasser und trocknet. Ausbeute: 9,4 g, entsprechend 81% d. Th.

Statt in Chlorbenzol kann die Reaktion auch in überschüssigem Phenol als Reaktionsmedium durchgeführt werden.

Der Farbstoff färbt Polyethylenterephthalatfasern und Cellulosetriacetatfasern in rotstichig gelben Tönen. Eine gleichwertige Gelbfärbung wird auf einem Gewebe aus Polycyclohexan-dimethylen-terephthalatfasern erhalten.

Beispiele 122—140

Analog wie in den Beispielen 22 und 121 beschrieben, werden die in Tabelle 3 aufgeführten Anthrachinonverbindungen hergestellt, die auf Geweben oder Gewirken aus Polyester-, Triacetat-, Polyamid-, Polyurethan- oder Polyolefinfasern die angegebenen Nuancen ergeben.

TABELLE 3

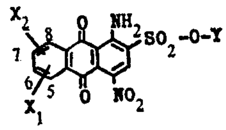

| Bsp.-Nr. | Y | $X_1$ | $X_2$ | Farbton |
|---|---|---|---|---|
| 122 | 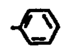 | 5-Cl | H | Gelb |
| 123 | 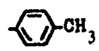 | 6-Cl | H | Gelb |
| 124 | 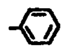 | 7-Cl | H | Gelb |
| 125 | 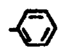 | 8-Cl | H | Gelb |
| 126 | 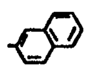 | 6-Cl | 7-Cl | Gelb |
| 127 | 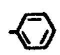 | 6-Cl | 7-Cl | Gelb |
| 128 | 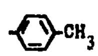 | 5-Cl | 8-Cl | Gelb |
| 129 | 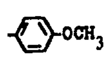 | 5-F | H | Gelb |
| 130 | 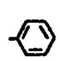 | 6-F | H | Gelb |
| 131 | 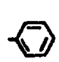 | 7-F | H | Gelb |
| 132 | 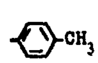 | 6-F | 7-F | Gelb |

TABELLE 3 (Fortsetzung)

| Bsp.–Nr. | Y | $X_1$ | $X_2$ | Farbton |
|---|---|---|---|---|
| 133 | 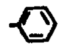 | 6-F | 7-F | Gelb |
| 134 | 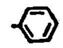 | 6-Br | H | Gelb |
| 135 | 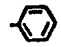 | 5-$NO_2$ | H | Gelb |
| 136 | 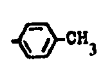 | 5-$NO_2$ | H | Gelb |
| 137 | 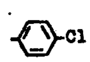 | 5-$NO_2$ | H | Gelb |
| 138 | 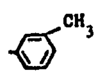 | 6-$NO_2$ | H | Gelb |
| 139 | 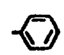 | 7-$NO_2$ | H | Gelb |
| 140 | 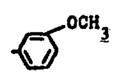 | 7-$NO_2$ | H | Gelb |

Beispiel 141

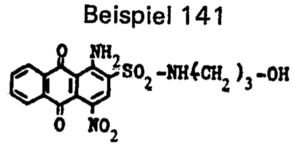

91,5 g 1-Amino-4-nitro-anthrachinon-2-sulfonsäurechlorid (Herstellung z.B. nach Beispiel 1) trägt man in 300 ccm Chlorbenzol ein und tropft unter Rühren 46,9 g 3-Hydroxypropylamin so zu, daß die Temperatur 30°C nicht übersteigt. Nach 30 Min. verdünnt man das Reaktionsgemisch mit 500 ccm Methanol, saugt nach weiteren 30 Min. ab, wäscht mit Methanol und dann mit Wasser und trocknet bei 60°C. Ausbeute: 81 g, entsprechend 80% d. Th.

Analyse: $C_{17}H_{15}N_3O_7S$ (405,4)

| | | | | |
|---|---|---|---|---|
| Berechnet: | C 50,37 | H 3,73 | N 10,37 | S 7,91 |
| Gefunden: | 50,5 | 3.8 | 10,6 | 7,9 |

Ersetzt man im vorstehenden Beispiel das Chlorbenzol durch die gleiche Menge Toluol, so kann

dasselbe Reaktionsprodukt in ähnlich guter Ausbeute und Reinheit isoliert werden. Auch 1,2-Dichlorbenzol, Nitrobenzol, Chloroform, 1,2-Dichlorethan, Dioxan, Tetrahydrofuran, Dimethylformamid, Dimethylsulfoxid, Sulfolan, Pyridin, Diglykolmonomethylether, Glykolmonomethylether, Glykoldimethylether, Aceton und Methylethylketon können mit demselben Erfolg als Lösungsmittel verwendet werden.

Statt mit überschüssigem Amin kann die Umsetzung auch mit der etwas mehr als stöchiometrisch erforderlichen Menge Amin in Gegenwart eines säurebindenden Mittels, z.B. nach folgender Vorschrift erfolgen:

In 40 ccm 1,2-Dichlorbenzol trägt man 2,1 g 3-Hydroxy-propylamin und 2,6 g trockenes Kaliumcarbonat ein und fügt dann 9,15 g 1-Amino-4-nitroanthrachinon-2-sulfonsäurechlorid so zu, daß die Temperatur 30°C nicht übersteigt. Nach 30 Min. verdünnt man das Reaktionsgemisch mit 40 ccm Methanol, saugt nach weiteren 30 Min. ab. wäscht mit Methanol und Wasser und trocknet bei 60°C. Ausbeute: 8,0 g, entsprechend 79% d. Th. desselben Produktes wie zuvor.

Ersetzt man im vorstehenden Beispiel das als säurebindendes Mittel verwendete Kaliumcarbonat durch Natriumcarbonat, Natriumhydrogencarbonat, Natriumhydroxid, Calciumoxid, Magnesiumoxid, Natriumacetat oder Kaliumacetat, so kann das Sulfonamid in ähnlich guter Ausbeute und Reinheit erhalten werden. Aehnlich gute Ergebnisse erzielt man auch mit den organischen Basen Trimethylamin, Triethylamin, Pyridin oder Benzyltrimethyl-ammoniumhydroxid.

Mit dem nach einer der vorstehenden Vorschriften hergestellten Farbstoff erzielt man auf synthetischen Fasermnaterialien aus Celluloseestern, Polyamiden, Polyurethanen, Polyacrylnitrilen und insbesondere Polyestern rotstichig gelbe bis orange Töne mit guten Echtheiten.

Beispiel 142

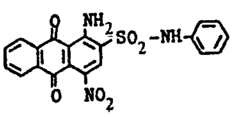

Zu der Suspension von 73,3 g 1-Amino-4-nitro-anthrachinon-2-sulfonsäurechlorid in 240 ccm 1,2-Dichlor-benzol tropft man 46,5 g Anilin so zu, daß die Temperatur 30°C nicht übersteigt. Nach 1-stündigem Rühren bei Raumtemperatur verdünnt man das Reaktionsgemisch mit 400 ccm Methanol, rührt nochmals 30 Min., saugt ab, wäscht mit Methanol und dann mit Wasser und trocknet bei 60°C. Ausbeute: 73,2 g, entsprechend 86% d. Th.

Ebenso glatt verläuft die Umsetzung in Chlorbenzol oder Toluol als Lösungsmittel und liefert das Produkt in praktisch gleicher Ausbeute und Reinheit.

Der Farbstoff ergibt auf synthetischen Fasermaterialien aus Celluloseestern, Polyamiden, Polyurethanen, Polyacrylnitrilen und insbesondere Polyestern rotstichig gelbe bis orange Töne mit guten Echtheiten.

Beispiele 143—333

Analog wie in den Beispielen 141 und 142 beschrieben, werden die in Tabelle 4 aufgeführten Anthrachinonverbindungen hergestellt, die auf Geweben oder Gewirken aus Polyester-, Polyamid-, Polyurethan- oder Polyolefinfasern die angegebenen Nuancen ergeben.

TABELLE 4

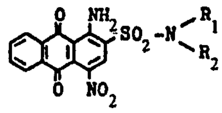

| Bsp.-Nr. | $R_1$ | $R_2$ | Farbton |
|---|---|---|---|
| 143 | H | H | rotst. Gelb |
| 144 | $CH_3$ | H | stark rotst. Gelb |
| 145 | $CH_3$ | $CH_3$ | rotst. Gelb |
| 146 | $C_2H_5$ | H | rotst. Gelb |
| 147 | $C_2H_5$ | $C_2H_5$ | rotst. Gelb |
| 148 | n-$C_3H_7$ | H | Orange |
| 149 | n-$C_3H_7$ | n-$C_3H_7$ | rotst. Gelb |
| 150 | i-$C_3H_7$ | H | Orange |
| 151 | i-$C_3H_7$ | i-$C_3H_7$ | Orange |
| 152 | n-$C_4H_9$ | H | Orange |
| 153 | n-$C_4H_9$ | n-$C_4H_9$ | rotst. Gelb |
| 154 | $CH_3$/$C_2H_5$ > CH– | H | rotst. Gelb |
| 155 | $(CH_3)_2CH-CH_2-$ | H | Orange |
| 156 | $(CH_3)_3C-$ | H | Orange |
| 157 | $CH_3-CH_2-C(CH_3)_2-$ | H | Orange |
| 158 | $CH_3-(CH_2)_5-$ | H | rotst. Gelb |
| 159 | $CH_3-(CH_2)_3-CH(C_2H_5)-CH_2-$ | H | rotst. Gelb |
| 160 | $CH_3-(CH_2)_9-$ | H | Orange |
| 161 | $Cl-CH_2-CH_2-$ | H | Orange |
| 162 | $H_2N-CH_2-CH_2-$ | H | Orange |
| 163 | $CH_3NH-CH_2-CH_2-$ | $CH_3$ | Orange |
| 164 | $(C_2H_5)_2N-(CH_2)_2-$ | H | rotst. Gelb |
| 165 | $CH_3-NH-(CH_2)_3-$ | H | rotst. Gelb |
| 166 | $(CH_3)_2N-(CH_2)_3-$ | H | Orange |
| 167 | $(C_2H_5)_2N-(CH_2)_3-$ | H | rotst. Gelb |
| 168 | $(C_4H_9)_2N-(CH_2)_3-$ | H | rotst. Gelb |
| 169 | $HO-(CH_2)_2-$ | H | rotst. Gelb |

TABELLE 4 (Fortsetzung)

| Bsp.–Nr. | $R_1$ | $R_2$ | Farbton |
|---|---|---|---|
| 170 | $CH_3O-(CH_2)_2-$ | H | Orange |
| 171 | $HO-(CH_2)_2-$ | $CH_3$ | rotst. Gelb |
| 172 | $HO-(CH_2)_2-$ | $HO-(CH_2)_2-$ | Orange |
| 173 | $HO-(CH_2)_3-$ | $CH_3$ | Orange |
| 174 | $CH_3O-(CH_2)_3-$ | H | Orange |
| 175 | $C_2H_5-O-(CH_2)_3-$ | H | Orange |
| 176 | $C_4H_9-O-(CH_2)_3-$ | H | Orange |
| 177 | $CH_3-CH(OH)-CH_2-$ | H | rotst. Gelb |
| 178 | $CH_3-CH(OH)-CH_2-$ | $CH_3-CH(OH)-CH_2-$ | Orange |
| 179 | $CH_2-CH(OH)-(CH_2)_2-$ | H | Orange |
| 180 | $HO-CH_2-C(CH_3)_2-$ | H | Orange |
| 181 | $CH_3O-CO-CH_2-$ | H | Orange |
| 182 | $CH_3O-CO-CH_2-$ | $CH_3$ | rotst. Gelb |
| 183 | $CH_3O-CO-(CH_2)_2-$ | H | rotst. Gelb |
| 184 | $NC-(CH_2)_2-$ | $CH_3$ | rotst. Gelb |
| 185 | $C_4H_9O-CO-CH_2-$ | H | Orange |
| 186 | $NC-(CH_2)_5-$ | H | rotst. Gelb |
| 187 | $CH_3O-CO-(CH_2)_{10}-$ | H | rotst. Gelb |
| 188 | 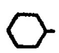 | H | rotst. Gelb |
| 189 | 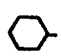 | $CH_3$ | Orange |
| 190 | 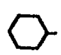 | $C_2H_5$ | Orange |
| 191 | 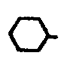 | 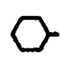 | Orange |
| 192 | 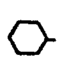 | $HO-(CH_2)_2-$ | rotst. Gelb |

TABELLE 4 (Fortsetzung)

| Bsp.-Nr. | $R_1$ | $R_2$ | Farbton |
|---|---|---|---|
| 193 | 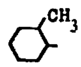 | H | rotst. Gelb |
| 194 | 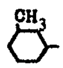 | H | Orange |
| 195 | 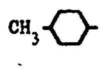 | H | Orange |
| 196 | 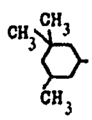 | H | rotst. Gelb |
| 197 | 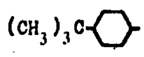 | H | rotst. Gelb |
| 198 | 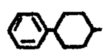 | H | rotst. Gelb |
| 199 | 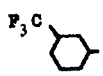 | H | Orange |
| 200 | 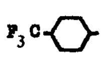 | H | Orange |
| 201 | 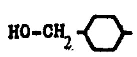 | H | Orange |
| 202 | 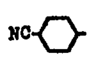 | H | Orange |
| 203 | 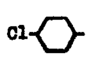 | H | Orange |
| 204 | 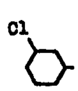 | H | Orange |

TABELLE 4 (Fortsetzung)

| Bsp.-Nr. | $R_1$ | $R_2$ | Farbton |
|---|---|---|---|
| 205 | 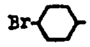 | H | Orange |
| 206 | 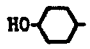 | H | Orange |
| 207 | 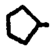 | H | rotst. Gelb |
| 208 | 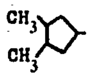 | H | Orange |
| 209 | 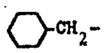 | H | rotst. Gelb |
| 210 | 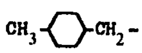 | H | Orange |
| 211 | 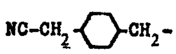 | H | Orange |
| 212 | 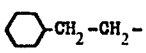 | H | Orange |
| 213 | 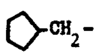 | H | Orange |
| 214 | 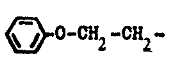 | H | rotst. Gelb |
| 215 | 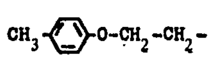 | H | Orange |
| 216 | 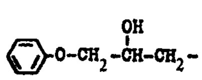 | H | Orange |
| 217 | $C_4H_9-NH-CH_2-CH_2-$ | H | rotst. Gelb |

TABELLE 4 (Fortsetzung)

| Bsp.-Nr. | $R_1$ | $R_2$ | Farbton |
|---|---|---|---|
| 218 | $CF_3$ | H | Orange |
| 219 | 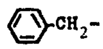 | H | Orange |
| 220 | 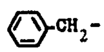 | $CH_3$ | rost. Gelb |
| 221 | 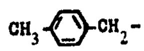 | H | rotst. Gelb |
| 222 | 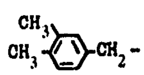 | H | rotst. Gelb |
| 223 | 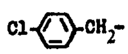 | H | rotst. Gelb |
| 224 | 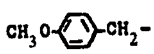 | H | Orange |
| 225 | 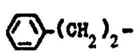 | H | rotst. Gelb |
| 226 | 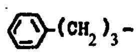 | H | Orange |
| 227 | 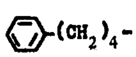 | H | Orange |
| 228 | 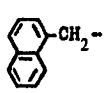 | H | Orange |
| 229 | 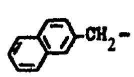 | H | Orange |

TABLE 4 (Fortsetzung)

| Bsp.–Nr. | $R_1$ | $R_2$ | Farbton |
|---|---|---|---|
| 230 | 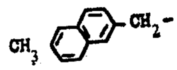 | H | rotst. Gelb |
| 231 | 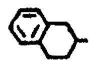 | H | rotst. Gelb |
| 232 | 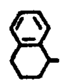 | H | Orange |
| 233 | 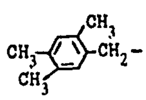 | H | Orange |
| 234 | 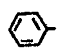 | $CH_3$ | Orange |
| 235 | 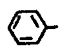 | $C_2H_5$ | Orange |
| 236 | 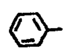 | n-$C_4H_9$ | rotst. Gelb |
| 237 | 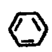 | $-Ch_2-CH_2-OH$ | Orange |
| 238 | 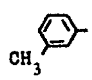 | n-$C_4H_9-$ | Orange |
| 239 | 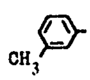 | $-CH_2-CH_2-OH$ | Orange |

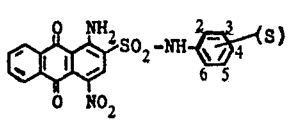

TABELLE 4 (Fortsetzung)

| Bsp.–Nr. | $(S)_n$ | Farbton |
|---|---|---|
| 240 | 2-Cl | Orange |
| 241 | 3-Cl | Orange |
| 242 | 4-Cl | Orange |
| 243 | 3,4-Di-Cl | rotst. Gelb |
| 244 | 3,5-Di-Cl | rotst. Gelb |
| 245 | 2-F | rotst. Gelb |
| 246 | 3-F | rotst. Gelb |
| 247 | 4-F | Orange |
| 248 | 4-Br | Orange |
| 249 | 3-$NO_2$ | Orange |
| 250 | 4-$NO_2$ | Orange |
| 251 | 3-$CF_3$ | Orange |
| 252 | 4-$CF_3$ | rotst. Gelb |
| 253 | 2-$CH_3$ | rotst. Gelb |
| 254 | 3-$CH_3$ | Orange |
| 255 | 4-$CH_3$ | Orange |
| 256 | 2-$CH_3$-5-Cl | Orange |
| 257 | 3-$CH_3$-4-Cl | rotst. Gelb |
| 258 | 2-Cl-5-$CF_3$ | Orange |
| 259 | 2,4-Di-Cl-5-$CH_3$ | Orange |
| 260 | 3-Cl-4-$CH_3$ | Orange |
| 261 | 2-$C_2H_5$ | rotst. Gelb |
| 262 | 4-$C_2H_5$ | rotst. Gelb |
| 263 | 3,4-Di-$CH_3$ | Orange |
| 264 | 2,4-Di-$CH_3$ | rotst. Gelb |
| 265 | 3,5-Di-$CH_3$ | rotst. Gelb |
| 266 | 3,5-Di-$CF_3$ | Orange |
| 267 | 2,5-Di-$CH_3$ | Orange |
| 268 | 2,4,5-Tri-$CH_3$ | rotst. Gelb |
| 269 | 4-tert.-Butyl | Orange |
| 270 | 4-Cyclopentyl | Orange |

TABELLE 4 (Fortsetzung)

| Bsp.–Nr. | $(S)_n$ | Farbton |
|---|---|---|
| 271 | 4-Cyclohexyl | Orange |
| 272 | $2\text{-}CH_3\text{-}4$-cyclohexyl | rotst. Gelb |
| 273 | 4-Phenyl | Orange |
| 274 | $3\text{-}N(CH_3)_2$ | Orange |
| 275 | $3\text{-}NH\text{–}CO\text{–}CH_3$ | Orange |
| 276 | $4\text{-}N(CH_3)_2$ | Orange |
| 277 | $4\text{-}N(C_3H_5)_3$ | rotst. Gelb |
| 278 | 4-NH-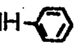 | rotst. Gelb |
| 279 | $2\text{-}OCH_3$ | rotst. Gelb |
| 280 | $2\text{-}OC_2H_5$ | Orange |
| 281 | 2-O-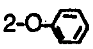 | rotst. Gelb |
| 282 | $2\text{-}OCH_3\text{-}5\text{-}Cl$ | rotst. Gelb |
| 283 | 3-OH | Orange |
| 284 | $3\text{-}OCH_3$ | Orange |
| 285 | $3\text{-}OC_2H_5$ | rotst. Gelb |
| 286 | 4-OH | rotst. Gelb |
| 287 | $4\text{-}OCH_3$ | rotst. Gelb |
| 288 | $4\text{-}OC_2H_5$ | rotst. Gelb |
| 289 | $4\text{-}O\text{-}(CH_2)_2\text{–}OH$ | Orange |
| 290 | $4\text{-}OC_4H_9$ | rotst. Gelb |
| 291 | 4-NH-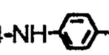$\text{-}OCH_3$ | rotst. Gelb |
| 292 | $4\text{-}SO_2\text{–}CH_3$ | Orange |
| 293 | $3\text{-}OH\text{-}4\text{-}CH_3$ | rotst. Gelb |
| 294 | $3\text{-}OCH_3\text{-}4\text{-}CH_3$ | Orange |
| 295 | $2\text{-}CH_3\text{-}4\text{-}OCH_3$ | rotst. Gelb |
| 296 | $2\text{-}CH_3\text{-}5\text{-}OCH_3$ | Orange |
| 297 | $2\text{-}OCH_3\text{-}4\text{-}Cl\text{-}5\text{-}CH_3$ | rotst. Gelb |
| 298 | $2\text{-}(SO_2\text{–}C_2H_5)\text{-}5\text{-}CF_3$ | Orange |

TABELLE 4 (Fortsetzung)

| Bsp.–Nr. | $(S)_n$ | Farbton |
|---|---|---|
| 299 | 2,4-Di-$OCH_3$ | rotst. Gelb |
| 300 | 2,5-Di-$OCH_3$ | rotst. Gelb |
| 301 | 2,5-Di-$OC_2H_5$ | rotst. Gelb |
| 302 | 2-$OCH_3$-5-($SO_2$–$CH_3$) | Orange |
| 303 | 2-COOH | rotst. Gelb |
| 304 | 3-COOH | Orange |
| 305 | 2-$COOCH_3$ | Orange |
| 306 | 2-$COOC_2H_5$ | Orange |
| 307 | 2-CO–$NH_2$ | rotst. Gelb |
| 308 | 3-CN | Orange |
| 309 | 4-COOH | Orange |
| 310 | 4-$COOC_2H_5$ | Orange |
| 311 | 4-CO–$NH_2$ | rotst. Gelb |
| 312 | 3,4-Di-CN | Orange |
| 313 | 2,4-Di-CN | Orange |
| 314 | 3-$SO_2NH_2$ | rotst. Gelb |
| 315 | 3-$SO_2$–NH–$(CH_2)_2$–OH | Orange |
| 316 | 4-CO–$CH_3$ | rotst. Gelb |
| 317 | 4-CO–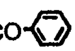 | rotst. Gelb |

TABELLE 4 (Fortsetzung)

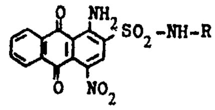

| Bsp.-Nr. | R | Farbton |
|---|---|---|
| 318 | 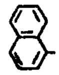 | Orange |
| 319 | 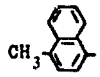 | Orange |
| 320 | 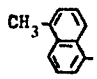 | Orange |
| 321 | 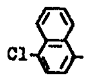 | rotst. Gelb |
| 322 | 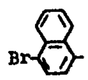 | rotst. Gelb |
| 323 | 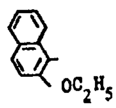 | rotst. Gelb |
| 324 | 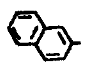 | Orange |
| 325 | 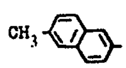 | Orange |
| 326 | 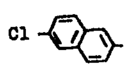 | Orange |
| 327 | 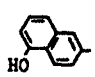 | Orange |
| 328 | 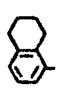 | Orange |

TABELLE 4 (Fortsetzung)

| Bsp.–Nr. | R | Farbton |
|---|---|---|
| 329 | 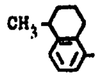 | Orange |
| 330 | 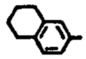 | rotst. Gelb |

TABELLE 4 (Fortsetzung)

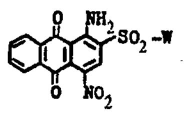

| Bsp.–Nr. | W | Farbton |
|---|---|---|
| 331 | 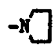 | Orange |
| 332 | 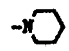 | Orange |
| 333 | 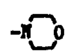 | Orange |

Beispiel 334

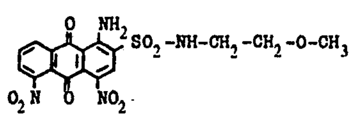

Zu der Mischung aus 8,2 g 1-Amino-4,5-dinitro-anthrachinon-2-sulfonsäurechlorid und 30 ccm trockenem Aceton tropft man 3,3 g 2-Methoxyethyl-amin so zu, daß die Temperatur nicht über 30°C steigt. Nach 30 Minuten bei Raumtemperatur verdünnt man das Reaktionsgemisch mit 30 ccm Methanol, rührt nochmals 30 Minuten, saugt dann ab, wäscht mit Methanol und Wasser und trocknet bei 60°C. Ausbeute: 8,1 g, entsprechend 90% d. Th.

Ebenso glatt verläuft die Umsetzung in Toluol oder Chlorbenzol als Lösungsmittel und liefert das Produkt in praktisch gleicher Ausbeute und Reinheit.

Der Farbstoff ergibt auf synthetischen Fasermaterialien aus Celluloseestern, Polyamiden, Polyurethanen, Polyacrylnitrilen und insbesondere Polyestern gelbe Töne mit guten Echtheiten.

Beispiele 335—363

Analog wie in den Beispielen 141, 142 und 334 beschrieben, werden die in Tabelle 5 aufgeführten Anthrachinonverbindungen hergestellt, die auf Geweben oder Gewirken aus Polyester-, Polyamid-, Polyurethan- oder Polyolefinfasern die angegebenen Nuancen ergeben.

TABELLE 5

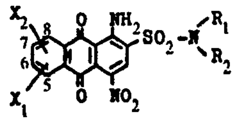

| Bsp.-Nr. | $R_1$ | $R_2$ | $X_1$ | $X_2$ | Farbton |
|---|---|---|---|---|---|
| 335 | H | H | 5-Cl | H | Orange |
| 336 | 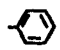 | H | 5-Cl | H | Orange |
| 337 | $-C_2H_5$ | $-C_2H_5$ | 6-Cl | H | rotst. Orange |
| 338 | 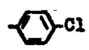 | H | 6-Cl | H | Orange |
| 339 | $-(CH_2)_3-OCH_3$ | H | 7-Cl | H | Orange |
| 340 | 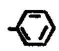 | H | 7-Cl | H | Orange |
| 341 | $-C_4H_9$ | H | 8-Cl | H | Orange |
| 342 | 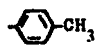 | H | 8-Cl | H | rotst. Orange |
| 343 | $-CH_2-CH_2-OH$ | H | 6-Cl | 7-Cl | Orange |
| 344 | 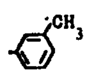 | H | 6-Cl | 7-Cl | Orange |
| 345 | 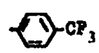 | H | 6-Cl | 7-Cl | Orange |
| 346 | 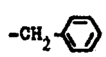 | H | 5-Cl | 8-Cl | Orange |
| 347 | $-(CH_2)_2-OCH_3$ | H | 5-Cl | 8-Cl | Orange |
| 348 | 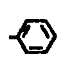 | H | 5-Cl | 8-Cl | Orange |
| 349 | 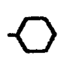 | H | 5-F | H | Orange |
| 350 | 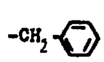 | H | 5-F | H | rotst. Orange |

TABELLE 5 (Fortsetzung)

| Bsp.-Nr. | $R_1$ | $R_2$ | $X_1$ | $X_2$ | Farbton |
|---|---|---|---|---|---|
| 351 | $-(CH_2)_3-OC_2H_5$ | H | 6-F | H | Orange |
| 352 | 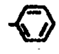 | H | 6-F | H | Orange |
| 353 | $-CH_2-CH_2-OH$ | H | 7-F | H | rotst. Orange |
| 354 | 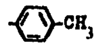 | H | 7-F | H | rotst. Orange |
| 355 | $-(CH_2)_3-OH$ | H | 8-F | H | Orange |
| 356 | $-CH_2-CH_2-OCH_3$ | H | 6-F | 7-F | Orange |
| 357 | $-(CH_2)_3-OCH_3$ | H | $5-NO_2$ | H | Gelb |
| 358 | 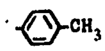 | H | $5-NO_2$ | H | Gelb |
| 359 | $-CH_3$ | $CH_3$ | $6-NO_2$ | H | Gelb |
| 360 | 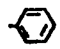 | H | $6-NO_2$ | H | Gelb |
| 361 | 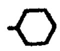 | H | $7-NO_2$ | H | Gelb |
| 362 | 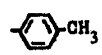 | H | $8-NO_2$ | H | Gelb |
| 363 | 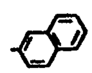 | H | $5-NO_2$ | H | Gelb |

*Färbebeispiele*

Beispiel 364

Mit 1 g Farbstoff des Beispiels 22, den man zuvor in Gegenwart von Dispergiermitteln in feine Verteilung gebracht hat, werden 100 g Polyethylenterephthalatfasern in 4 l Wasser in Gegenwart von 15 g o-Kresotinsäuremethylester als Carrier 2 Stunden bei 100°C und pH 4,5 gefärbt. Man gewinnt eine rotstichig gelbe Färbung, die durch gute Wasch-, Thermofixiert-, Reib- und Lichtechtheiten ausgezeichnet ist. Eine ähnliche Färbung wird erhalten, wenn man als Polyesterfasern solche aus 1,4-Bis-(hydroxymethyl)-cyclohexan und Terephthalsäure verwendet.

Ebenso lassen sich auch die nach Beispiel 23 bis 140 erhaltenen Farbstoffe färben.

Beispiel 365

10 g Strangmaterial aus Polyethylenglykolterephthalat werden in einer Flotte von 600 ccm Wasser, 0,1 g in feiner Verteilung vorliegendem Farbstoff des Beispiels 141, 3,4 g eines Gemisches aus o-, m-, p-Kresotinsäuremethylester und 0,6 g einer Mischung aus Gleichen Teilen Aralkylsulfonat und eines nicht ionogenen Polyglykolethers nach Zusatz von Schwefelsäure bis zur Einstellung eines pH-Wertes von 4,5 2 Stunden bei 96 bis 98°C gefärbt. Anschließend wird gespült und getrocknet. Die Reibechtheit kann verbessert werden, wenn man in einem schwach kockenden Bad, das auf 1000 ccm Wasser 5,5 g Natronlauge von 38° Bé 2 g Natriumdithionit und 1 g eines Polyglykolethers eines Fettsäureamids enthält, 10 bis 30 Minuten nachbehandelt. Man erhält eine orange Färbung von guten Echtheitseigenschaften, insbesondere einer sehr guten Sublimierechtheit.

Ebenso lassen sich auch die nach Beispiel 22—140 und 142 bis 363 erhaltenen Farbstoffe färben.

Beispiel 366

Mit 1 g des in Beispiel 25 genannten, zuvor mit den üblichen Hilfsmitteln in feine Verteilung gebrachten Farbstoffes werden 100 g Polyesterfasern (Polyethylenterephthalat) in 3 l Wasser während 1 Stunde bei 125—130°C unter Druck gefärbt. Man erhält eine gelbe Färbung von guten Echtheiten.

Ebenso lassen sich auch die nach Beispiel 22 bis 24 und 26 bis 140 erhaltenen Farbstoffe färben.

Beispiel 367

10 g eines Gewebes aus Polyethylenglykolterephthalat werden in einer Flotte vom pH 4,5 aus 400 ccm Wasser und 0,1 g in feinster Verteilung vorliegendem Farbstoff des Beispiels 142, sowie 0,3 g einer Mischung aus gleichen Teilen Aralkylsulfonats und eines nicht ionogenen Polyglykolethers 2 Stunden bei 120 bis 130° gefärbt. Nach dem Spüllen und Trocknen erhält man eine rotstichig gelbe Färbung von guten bis sehr guten Echtheitseigenschaften, insbesondere einer sehr guten Sublimierechtheit. Ebenso lassen sich auch die nach Beispiel 22 bis 141 und 143 bis 363 erhaltenen Farbstoffe färben.

Beispiel 368

20 g Cellulose-2$^1/_2$-acetatfasern werden in einer Flotte aus 600 ml Wasser, 1 g Marseiller Seife und 0,2 g des in Beispiel 172 genannten, in feine Verteilung gebrachten Farbstoffes 1 Stunde bei 75°C gefärbt. Man erhält eine Orangefärbung mit guten Reib-, Licht- und Waschechtheiten.

Ebenso lassen sich auch die anderen genannten Farbstoffe färben.

Beispiel 369

Mit 1 g des Farbstoffes aus Beispiel 61 der zuvor unter Verwendung der hierfür üblichen Hilfsmittel in feine Verteilung gebracht wurde, 6 g Fettalkoholsulfonat und 3 l Wasser wird ein Färbebad bereitet, in dem 100 g Cellulosetriacetatfasern 1 Stunde bei 100°C gefärbt werden. Man erhält eine rotstichig gelbe Färbung von sehr guter Wasch-, Thermofixier-, Reib- und Lichtechtheit.

Ebenso lassen sich auch die anderen genannten Farbstoffe färben.

Beispiel 370

Mit 1 g des in Beispiel 171 genannten Farbstoffes, den man zuvor nach den üblichen Methoden in feine Verteilung gebracht hat, werden 100 g Polyamidgewebe in 4 l Wasser während 1 Stunden bei 100°C gefärbt. Das Gewebe wird anschließend warm und kalt gespült und getrocknet. Man erhält eine rotstichige Gelbfärbung von guten Echtheiten. Anstelle von Polyamidfasern lassen sich mit gleichem Erfolg Polyurethanfasern verwenden.

Ebenso lassen sich auch die anderen genannten Farbstoffe färben.

Beispiel 371

Ein Gewebe aus Polyesterfasern (Polyethylenterephthalat) wird auf dem Foulard mit einer Flotte imprägniert, die im Liter 20 g Farbstoff des Beispiels 174 enthält, den man vorher in Gegenwart von Dispergiermitteln in feine Verteilung gebracht hat. Das Gewebe wird auf eine Gewichtszunahme von 70% abgequetscht und in einem Schwebedüsentrockner oder Trockenschrank bei 80—120°C getrocknet. Anschließend wird das Gewebe in einem Spannrahmen oder Düsenhotflue ca. 45 Sekunden bei 190—220°C mit heißer Luft behandelt, hiernach gespült, eventuell reduktiv nachbehandelt, gewaschen, gespült und getrocknet. Die reduktive Nachbehandlung zwecks Entfernung von oberflächlich an den Fasern anhaftenden Farbstoffanteilen kann so erfolgen, daß man mit dem Gewebe bei 25°C in eine 3—5 cm$^3$/l Natronlauge von 38° Bé und 1—2 g/l Natriumdithionit (konz.) enthaltende Flotte eingeht, innerhalb von ca. 15 Minuten auf 70°C erhitzt und weitere 10 Minuten bei 70°C beläßt. Anschließend wird heiß gespült, mit 2—3 cm$^3$/l 85%iger Ameisensäure bie 50°C angesäuert, gespült und getrocknet. Man erhält eine orange Färbung, die sich durch gute Echtheiten auszeichnet.

Eine ähnliche Färbung wird gewonnen, wenn man anstelle von Polyethylenterephthalatfasern Polyesterfasern aus 1,4-Bis-(hydroxymethyl)-cyclohexan und Terephthalsäure verwendet. In ähnlicher Weise erhält man eine rotstichige Gelbfärbung, wenn man anstelle von Polyethylenterephthalatfasern Cellulosetriacetatfasern einsetzt und die Thermosolierung bei 215°C durchführt oder wenn man Polyamid- oder Polyurethanfasern verwendet und die Thermosolierung bei 190—215°C vornimmt.

Ebenso lassen sich auch die nach Beispiel 22 bis 173 und 175 bis 363 erhaltenen Farbstoffe färben.

Beispiel 372

Ein vorgereinigtes und thermofixiertes Gewebe aus Polyethylenterephthalat wird mit einer aus folgenden Komponenten bestehenden Paste bedruckt:

20 g Farbstoff, erhalten nach Beispiel 142 in feiner Verteilung,
520 g Wasser,
450 g Kristallgummi 1:2,
10 g Kresotinsäuremethylester.

Anstelle von Kristallgummi kann auch eine Alginat-Verdickung Verwendung finden. Die bedruckte und getrocknete Ware wird zur Farbstoff-Fixierung bei 200°C mit Heißluft behandelt bzw. bei 190—200°C über einen Hochleistungsspannrahmen oder durch einen Kondensationsapparat gefahren. Die Einwirkungsdauer liegt bei 30—60 Sekunden. Der erhaltene fixierte Druck wird anschließend kalt gespült, mit 1—2 g/l anionaktivem Waschmittel bei 70—80°C ca. 10 Minuten geseift, erst heiß und dann kalt gespült und getrocknet. Man erhält einen klaren orangefarbigen Druck von guten Echtheiten.

In ähnlicher Weise gewinnt man einen rotstichig gelben Druck, wenn man anstelle von Polyethylen-terephthalatfasern Cellulosetriacetat-, Polyamid- oder Polyurethanfasern einsetzt.

Ebenso lassen sich die nach beispiel 22 bis 141 und 143 bis 363 erhaltenen Farbstoffe färben.

**Patentansprüche**

1. Anthrachinonfarbstoffe der Formel

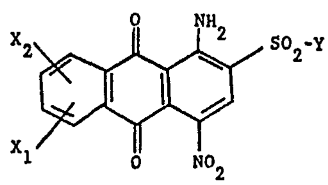

I

in welcher

Y eine Gruppe der Formeln —O—R oder

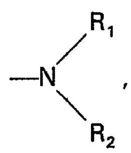

,

R gegebenenfalls substituiertes Aryl,

$R_1$ und $R_2$ Wasserstoff, gegebenenfalls substituiertes Alkyl, Cycloalkyl, Aralkyl oder Aryl, oder — gemeinsam — zusammen mit dem bindenden Stickstoffatom einen Ring und

$X_1$ und $X_2$ Wasserstoff, Halogen oder Nitro bedeuten.

2. Anthrachinonfarbstoffe gemäß Anspruch 1, dadurch gekennzeichnet, daß R einen durch 1 bis 3 Reste aus der Reihe $C_1$- bis $C_4$-Alkyl, $C_1$- bis $C_4$-Alkoxy, Halogen, wie insbesondere Fluor, Chlor, Brom, Methylmercapto, Nitro, Trifluormethyl, $C_5$- bis $C_6$-Cycloalkyl, Phenol, Acetyl, Benzoyl, Carbomethoxy oder Carboxyl substituierten Phenylrest oder einen gegebenenfalls durch Methyl, Chlor oder Brom substituierten Naphthyl- oder 5,6,7,8-Tetrahydronaphthylrest bedeutet.

3. Verfahren zur Herstellung von Anthrachinonfarbstoffen gemaß Anspruch 1, dadurch gekennziechnet, daß man Anthrachinonverbindungen der Formel

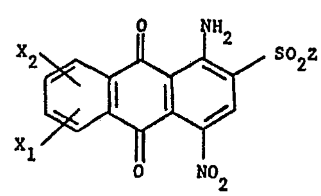

II

in welcher

$X_1$ und $X_2$ die in Anspruch 1 genannte Bedeutung haben und

Z für Chlor oder Brom steht,

mit Phenolen der Formel R—OH oder mit Aminen der Formel

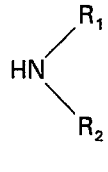

in welchen R, $R_1$ und $R_2$ die in Anspruch 1 genannte Bedeutung haben, umsetzt.

4. Verfahren zum Färben und Bedrucken von synthetischen Materialien, dadurch, gekennzeichnet, daß man Farbstoffe gemäß Anspruch 1 verwendet.

5. Anthrachinonverbindungen der Formel

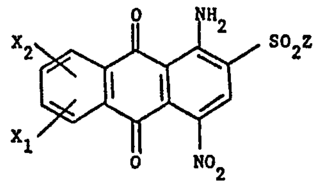

II

in welcher $X_1$, $X_2$ und Z in Anspruch 1 bzw. 3 genannte Bedeutung haben.

6. Anthrachinonverbindungen gemäß Anspruch 5, dadurch gekennzeichnet, daß Z für Chlor steht.

7. Verfahren zur Herstellung von Anthrachinonverbindungen gemäß Anspruch 5, dadurch gekennzeichnet, daß man Anthrachinonsulfonsäuren der Formel

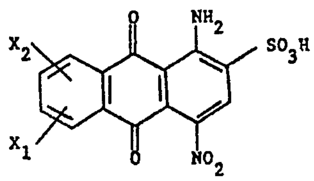

in welcher $X_1$ und $X_2$ die in Anspruch 1 genannte Bedeutung haben, mit entsprechenden anorganischen Säurehalogeniden umsetzt.

**Revendications**

1. Colorants anthraquinoniques répondant à la formule

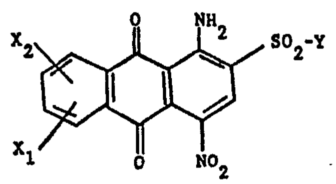

I

dans laquelle:

Y représente un groupe de formule —O—R ou

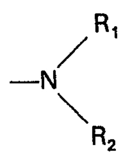

R représente un groupe aryl éventuellement substitué,

$R_1$ et $R_2$ représent l'hydrogène, des groupes alkyle, cycloalkyle, aralkyle ou aryle éventuellement substitués, ou forment ensemble, et avec l'atome d'azote qui les relie, un cycle, et

$X_1$ et $X_2$ représentent l'hydrogène, des halogènes ou des groupes nitro.

2. Colorants anthraquinoniques selon la revendication 1, caractérisés en ce que R représente un reste phényle portant 1 à 3 substituants pris dans la classe formée par les groupes alkyle en $C_1$—$C_4$, alcoxy en $C_1$—$C_4$, les halogènes et, en particulier, le fluor, le chlore, le brome, les groupes méthylmercapto, nitro, trifluorométhyle, cycloalkyle en $C_5$—$C_6$, phényl, acétyle, benzoyle, carbométhoxy ou carboxyle, ou un reste naphthyle ou 5,6,7,8-tétrahydronaphthyle portant éventuellement des substituants méthyle, chloro ou bromo.

3. Procédé de préparation des colorants anthraquinoniques selon la revendication 1, caractérisé en ce que l'on fait réagir des composés anthraquinoniques répondant à la formule

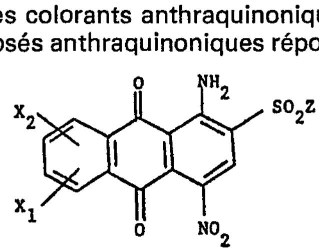

II

dans laquelle:

$X_1$ et $X_2$ ont les significations indiquées dans la revendication 1, et

Z représente le chlore ou le brome,

avec des phénols de formule R—OH ou des amines de formule

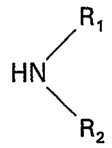

dans lesquelles R, $R_1$ et $R_2$ ont les significations indiquées dans la revendication 1.

4. Procédé pour la teinture et l'impression de matières synthétiques, caractérisé en ce que l'on utilise des colorants selon la revendication 1.

5. Composés anthraquinoniques répondant à la formule

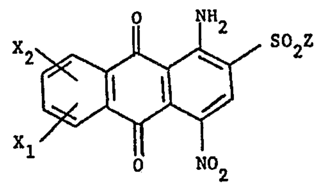

II

dans laquelle $X_1$, $X_2$ et Z ont les significations indiquées dans la revendication 1 ou 3.

6. Composés anthraquinoniques selon la revendication 5, caractérisé en ce que Z représente la chlore.

7. Procédé de préparation des composés anthraquinoniques selon la revendication 5, caractérisé en ce que l'on fait réagir les acides anthraquinone-sulfoniques répondant à la formule

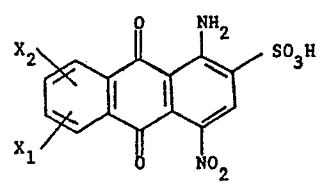

dans laquelle $X_1$ et $X_2$ ont les significations indiquées dans la revendication 1, avec les halogénures d'acides minéraux correspondants.

## Claims

1. Anthraquinone dyestuffs of the formula

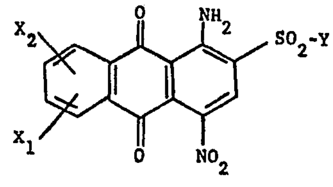

I

in which

Y denotes a group of the formulae —O—R or

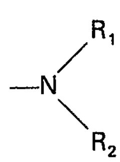

,

R denotes optionally substituted aryl,

$R_1$ and $R_2$ denote hydrogen or optionally substituted alkyl, cycloalkyl, aralkyl or aryl, or — together — with the bonding nitrogen atom, a ring and

$X_1$ and $X_2$ denote hydrogen, halogen or nitro.

2. Anthraquinone dyestuffs according to Claim 1, characterised in that R denotes a phenyl radical which is substituted by 1 to 3 radicals from the series $C_1$- to $C_4$-alkyl, $C_1$- to $C_4$-alkoxy, halogen, such as,

in particular, fluorine, chlorine or bromine, methylmercapto, nitro, trifluoromethyl, $C_5$- to $C_6$-cycloalkyl, phenyl, acetyl, benzoyl, carbomethoxy or carboxyl or a naphthyl radical or 5,6,7,8-tetrahydronaphthyl radical which is optionally substituted by methyl, chlorine or bromine.

3. Process for the preparation of anthraquinone dyestuffs according to Claim 1, characterised in that anthraquinone compounds of the formula

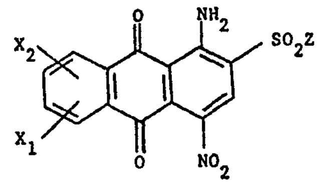

II

in which

$X_1$ and $X_2$ have the meaning given in Claim 1 and

Z represents chlorine or bromine,

are reacted with phenols of the formula R—OH or with amines of the formula

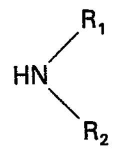

in which

R, $R_1$ and $R_2$ have the meaning given in Claim 1.

4. Process for dyeing and printing synthetic materials, characterised in that dyestuffs according to Claim 1 are used.

5. Anthraquinone compounds of the formula

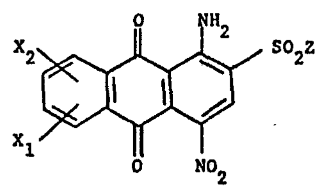

II

in which

$X_1$, $X_2$ and Z have the meaning given in Claim 1 and 3.

6. Anthraquinone compounds according to Claim 5, characterised in that Z represents chlorine.

7. Process for the preparation of anthraquinone compounds according to Claim 15, characterised in that anthraquinonesulphonic acids of the formula

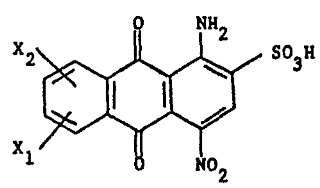

in which

$X_1$ and $X_2$ have the meaning given in Claim 1,

are reacted with corresponding inorganic acid halides.